# EUROPEAN PATENT SPECIFICATION

(11) **EP 2 531 528 B1**
(45) Date of publication and mention of the grant of the patent: **18.03.2015**
(21) Application number: 11740173.7
(22) Date of filing: 18.01.2011
(51) Int. Cl.: C08B 1/00, C12P 19/00, C13B 10/00, C12P 7/10, C12P 13/02, C08B 30/12, C13K 1/02, C12P 7/02, C12P 7/24, C13K 13/00, C08L 1/02, C08L 97/00

(54) **METHOD OF PRODUCING SUGARS USING A COMBINATION OF ACIDS TO SELECTIVELY HYDROLYZE HEMICELLULOSIC AND CELLULOSIC MATERIALS**
VERFAHREN ZUR HERSTELLUNG VON ZUCKERN MITTELS EINER KOMBINATION AUS SÄUREN ZUR SELEKTIVEN HYDROLYSIERUNG VON HEMIZELLULOSE- UND ZELLULOSEMATERIALIEN
PROCÉDÉ DE PRODUCTION DE SUCRES À L'AIDE D'UNE COMBINAISON D'ACIDES POUR HYDROLYSER SÉLECTIVEMENT DES MATÉRIAUX HÉMICELLULOSIQUES ET CELLULOSIQUES

(30) Priority: 03.02.2010 US 300853 P
(43) Date of publication of application: 12.12.2012
(73) Proprietor: Archer Daniels Midland Company, Decatur, IL 62526 (US)
(72) Inventor: BINDER, Thomas P., Decatur Illinois 62522 (US); BLOOM, Paul D., Forsyth, Illinois 62535 (US); DOANE, Perry H., Decatur Indiana 46733 (US); MA, Chicheng, Forsyth Illinois 62535 (US)
(74) Representative: Von Kreisler Selting Werner - Partnerschaft von Patentanwälten und Rechtsanwälten mbB
(86) International application number: PCT/US2011/021518
(87) International publication number: WO 2011/097065

(56) References cited:
- US-A- 5 536 325
- US-A1- 2005 136 520
- US-A1- 2007 083 039
- US-A1- 2008 227 162

## Description

This invention concerns an improved process for accomplishing the hydrolysis of materials containing cellulose and hemicellulose, and especially of lignocellulosic biomasses for further use in the synthesis of chemicals or the preparation of biobased fuels or fuel additives.

The use of biomass - of materials whose carbon content is of biological rather than fossil origin - for providing chemicals and fuel products presently derived from fossil-origin materials such as petroleum, or for providing acceptable biobased, functional alternatives to such chemicals and fuel products, has increasingly become a focus of research and development investment and effort in recent years as supplies of fossil-origin materials have been compromised or been more difficult or expensive to acquire and use.

Certain chemical and fuel product replacements or alternatives are already produced on a large, commodity scale from biomasses. For the liquid fuel products area, for instance, ethanol and biodiesel (fatty acid alkyl esters) have heretofore been produced on a commodity scale from corn or other grains and from sugar cane (for ethanol) and from various vegetable oils and fats (for biodiesel).

It has been long recognized, though, that it would be preferable to be able to make suitable liquid fuels and fuel additives from lignocellulosic biomasses containing typically 6 percent or more of acid detergent insoluble lignin (on a dry weight basis) and which are not used as food products, or which can be harvested or sourced and used without materially adversely affecting land use patterns and behaviors (for example, deforestation to produce additional soy, corn or like crops). A number of non-food, lignocellulosic biomasses might be contemplated of this character, including, for example, purpose-grown non-food biomass crops (such as grasses, sweet sorghum, fast growing trees), or more particularly wood wastes (such as prunings, wood chips, sawdust) and green wastes (for instance leaves, grass clippings, vegetable and fruit wastes). It has been estimated in addition as to lands already under cultivation for food crops or other purposes that about three quarters of the biomass generated is waste, so that whether the biomass in question is waste in the production of a food crop or some other crop to which land has been devoted in cultivation or arises from sources unconnected to any cultivated crop, it would seem with the abundance of lignocellulosic feeds available that the various chemical and fuel products we require that *could* be made starting with lignocellulosic biomasses, *should* in fact be capable of being made economically.

Unfortunately, the truth of the matter is that there are a number of practical, real-world difficulties that must be overcome in order for this proposition to hold true. A first difficulty arises from the very different characteristics of the various components comprising lignocellulosic biomasses.

In this regard, as is true of fossil-based materials such as petroleum, the practical, real-world capability of producing the full range of commodity chemicals and fuel product replacements or alternatives that are or will be needed, on the scale and with the qualities, economy and efficiency that are needed, depends to an extent on how effectively and efficiently the feedstock - lignocellulosic biomass - can be fractionated into its component parts and on how effectively and efficiently these component parts can in turn be further processed to yield the desired commodity chemicals and fuel product replacements or alternatives.

With respect to the present invention, lignocellulosic biomasses are comprised mainly of cellulose, hemicellulose and lignin fractions, with cellulose being the largest of these three components. Cellulose derives from the structural tissue of plants, and consists of long chains of beta glucosidic residues linked through the 1,4 positions. These linkages cause the cellulose to have a high crystallinity and thus a low accessibility to the enzymes or acid catalysts which have been suggested for hydrolyzing the cellulose to C6 sugars or hexoses for further processing. Hemicellulose by contrast is an amorphous heteropolymer which is easily hydrolyzed, while lignin, an aromatic three-dimensional polymer, is interspersed among the cellulose and hemicellulose within a plant fiber cell and lends itself to still other process options.

Parenthetically in regards to the lignin fraction, the materials understood as encompassed within the term "lignin" and the method by which lignin content has been correspondingly quantified in a biomass have historically depended on the context in which the lignin content has been considered, "lignin" lacking a definite molecular structure and thus being determined empirically from biomass to biomass. In animal science and agronomy, in considering the digestible energy content of lignocellulosic biomasses, for example, the amount of lignin in a given biomass has more commonly been determined using an acid detergent lignin method (Goering and Van Soest, Forage Fiber Analyses (Apparatus, Reagents, Procedures, and Some Applications), Agriculture Handbook No. 379, Agricultural Research Service, United States Dept of Agriculture (1970); Van Soest et al., "Methods for Dietary Fiber, Neutral Detergent Fiber, and Nonstarch Polysaccharides in Relation to Animal Nutrition", J. Dairy Sci., vol. 74, pp 3583-3597 (1991)). In the paper and pulp industry, by contrast, the amount of lignin in a given biomass has been conventionally determined by the Klason lignin method (Kirk and Obst, "Lignin Determination", Methods in Enzymology, vol 16, pp.: 89-101 (1988)). For purposes of the present invention, we are especially concerned with those lignocellulosic biomasses having at least a lignin content consistent with mature temperate grasses having relatively low nutritive value for ruminants and which consequently are diverted to other uses in the main, such grasses typically being characterized by 6% or more of acid detergent insoluble materials (on a dry weight basis).

Because of the differences in the cellulosic, hemicellulosic and lignin fractions of biomass, as well as considering other lesser fractions present in various biomasses to different degrees, as related in United States Patent No. 5,562,777 to Farone et al., "Method of Producing Sugars Using Strong Acid Hydrolysis of Cellulosic and Hemicellulosic Materials", a number of processes have been developed or proposed over the years to fractionate lignocellulosic biomasses and hydrolyze the cellulosic and hemicellulosic fractions.

Fundamentally both biological and non-biological processes have been disclosed, with the oldest and best known non-biological methods of producing sugars from cellulose involving acid hydrolysis, most commonly sulfuric acid-based hydrolysis using a dilute acid approach, a concentrated acid approach or a combination of the two. The '777 patent to Farone et al. describes the advantages and disadvantages of the various sulfuric acid-based processes then known to the art, and suggests a further variation using strong acid/sulfuric acid hydrolysis and employing one or more iterations of a combination of a decrystallization step wherein the biomass (and/or including the solids left from the decrystallization step in a previous iteration) is mixed with a 25-90 percent sulfuric acid solution to solubilize a portion of the biomass, then the acid is diluted to between 20 and 30 percent and the mixture heated to preferably between 80 and 100 degrees Celsius for a time to solubilize the cellulosic fraction and any hemicellulosic material that had not been hydrolyzed.

A further difficulty arises from the fact that, as mentioned previously, it has been estimated that about three quarters of the biomass generated on cultivated lands and grasslands is waste. While these waste biomasses represent a vast potential resource for the production of various biobased chemical and fuel products, the means for making and distributing such chemical and fuel products as an alternative to petroleum-based materials - as well as the end use markets for such chemical and fuel products - are generally not located where (or near where) the waste biomasses are produced or generated. The same observation holds true of the purpose-grown, non-food biomass crops that have been proposed or are being considered. Consequently, one seeking to make use of the various lignocellulosic biomasses available for making chemicals, fuels and fuel alternatives or additives has heretofore been faced either with constructing a number of smaller capacity, greenfield chemical plants or refineries near where the biomasses are collected or generated but remote from distribution channels and customers, with constructing a number of processing facilities to process the biomasses to an intermediate product or products (sugar syrup or sugar alcohols, for example) and then shipping the intermediate product or products to a further chemicals or fuels manufacturing and/or refining facility located (in the main) closer to distribution channels and customers, or with shipping the biomasses from the places where these have been collected or generated to a central facility wherein the biomasses will be processed from the beginning.

With respect to the processing and associated transportation/logistical/distribution and sales options just mentioned, one difficulty inherent to the use of lignocellulosic biomass feeds but not encountered with petroleum-based feedstocks, derives from the fact that biomasses arise from living matter. For example, one of the challenges to using corn stover as a biomass feed, whether for producing ethanol by fermentation or other chemicals, biobased fuels or fuel alternatives that can be made starting from a corn stover feed (e.g., diols and polyols, acrylates, hydroxymethylfurfural and other furanics, levulinates, epichlorohydrin), has been determining how much of the stover should be collected, as well as how it should be chopped, packaged or bundled, stored and transported to provide a consistent biomass feed with the right qualities; in this regard, as with any transformative process, consistency of the feedstock is always a concern, and as biomass is derived from living organisms the quality of collected biomass is inherently somewhat changeable so that proximity to storage, transport and processing facilities has needed to be factored into biomass selection.

The present invention provides methods for processing lignocellulosic biomasses in ways that can address and overcome some or all of the above-mentioned difficulties. In particular, the present invention concerns a method for producing sugars using a combination of acids to hydrolyze hemicellulosic and cellulosic materials in biomass, said combination of acids namely comprising a first, weak organic acid (such as acetic acid or formic acid) for providing a pentose product or stream from hydrolyzing hemicellulosic materials in the biomass on a batchwise, semi-continuous or continuous basis, and a second, strong mineral acid (such as sulfuric acid) for providing a hexose product or stream from hydrolyzing cellulosic materials in the biomass.

In a first aspect - after optionally preprocessing the biomass to isolate a component higher in protein that may be desirable for animal feed or fertilizer (by mechanically breaking down the biomass and by air classification, as one example) and/or to isolate a component or components which have a comparatively high content of a species or material that will be more difficult to remove downstream and that may interfere with or make intended downstream conversions more difficult and/or may adversely affect the contemplated products from further processing (for example, nitrogen compounds, sulfur compounds, higher ash components) - the first, weak organic acid is applied to the biomass, near a collection point for the biomass, under conditions (for example, in terms of acid concentrations, temperatures, pressures and residence times) sufficient to depolymerize hemicellulosic materials and solubilize lignins in the biomass. The "cooked" acidified biomass is then dried to remove water therefrom to an extent whereby the dried solids are pelletized for shipment to a central facility. Then, at the central facility, pelletized, weak acid-processed biomass is washed with a solvent or solvent mixture which is effective for separating the solubilized and depolymerized hemicelluloses and lignins from a cellulosic fraction of the biomass, and then the cellulosic fraction is contacted with the second, strong mineral acid (or acids) under conditions suited to providing a hexose product or stream. Preferably, the first, weak organic acid is applied to the biomass in a vapor form at elevated temperatures, in part to reduce the drying load prior to the pelletization step.

In a related, second aspect, the first, weak organic acid - which will be understood in common with references to the second, strong mineral acid to embrace both a single acid so characterized as well as combinations of acids so characterized - is applied to the biomass (or that portion of the biomass left after optionally preprocessing the biomass as a whole, as described above) under suitable conditions for hydrolyzing hemicellulosic materials in the biomass, and then the weak acid-processed biomass is washed with a solvent or solvent mixture to separate the pentose product or stream (from hydrolyzing the hemicellulosic materials in the biomass) from a remaining predominantly cellulosic solids fraction. The cellulosic solids fraction is then dried, pelletized and shipped to a central location for further processing with the second, strong mineral acid, while solubilized hemicellulosic materials and lignins are retained and optionally further processed at the first site, near a collection point for the biomass. Generally, this further processing will include at least a water wash, to effect a separation of water-insoluble lignins as a solids residue from the pentose product or stream resulting from hydrolysis of hemicellulosic materials in the biomass. It is also understood that pending local market conditions the "cooked" acidified biomass or cellulosic solids could be made available as a feed ingredient.

In a further aspect, not part of the invention, the first, weak organic acid is applied to the biomass (or to the remainder after optional preprocessing of the biomass, again), but then the weak acid-processed biomass is washed with a solvent or solvent mixture to separate the solubilized hemicellulosic materials and lignins from a predominantly cellulosic solids fraction, followed by contacting the cellulosic solids fraction with a strong mineral acid or acids, with the weak acid and strong acid hydrolyses occurring at the same location. Preferably, the first, weak organic acid is again applied to the biomass in a hot vapor form.

Dependent on conventional siting considerations such as the logistics involved in moving either collected biomass to a central processing facility or for getting derivative products to the markets and customers served by those products, access to utilities, labor and other process inputs etc., those skilled in the art will appreciate that in this last general conception of the inventive process the location in question may ideally be near a grouping of collection points for the biomass, or may more preferably be at a central location relative to the biomass sources to be drawn upon for the facility and convenient to the markets and customers to be served by the facility.
Figure 1 is a schematic drawing illustrating a process according to the present invention, according to the first aspect.
Figure 2 is a schematic drawing illustrating a process according to the present invention, according to the second aspect.
Figure 3 is a schematic drawing illustrating a process, according to the further aspect described above, not part of the invention.

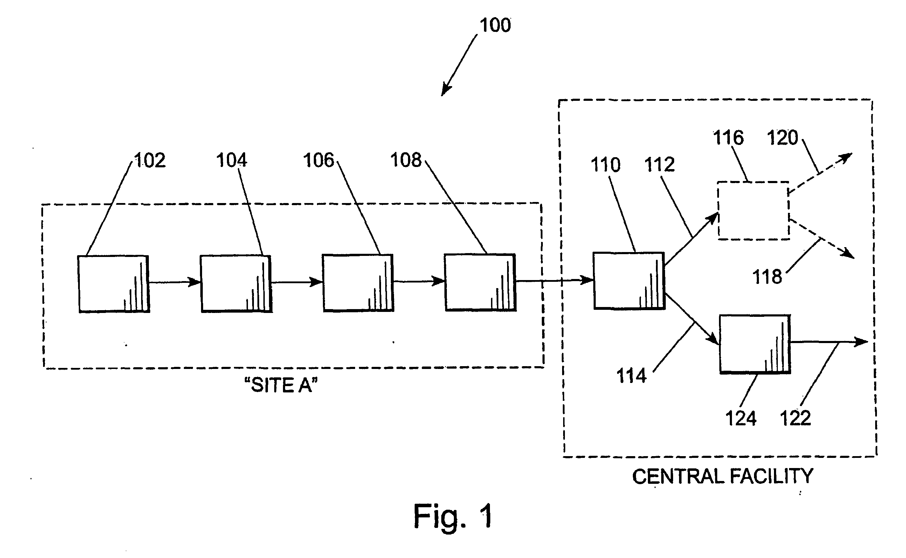

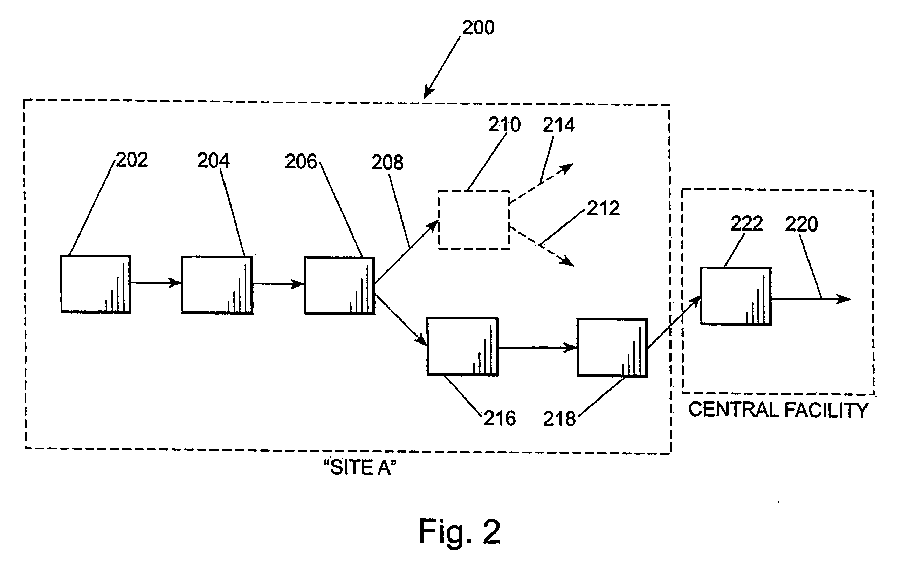

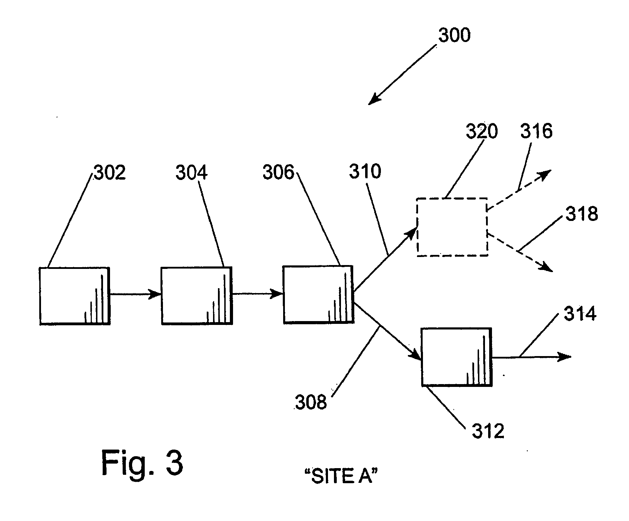

The present, combined acids process for producing sugars by hydrolyzing hemicellulosic and cellulosic materials in lignocellulosic biomass is more readily understood by reference to the accompanying drawings, by which the present invention is shown in a first aspect or general embodiment 100 (Figure 1), and in a second aspect or general embodiment 200 (Figure 2). General embodiment 300 (Figure 3) is not part of the invention.

Turning first to Figure 1, a lignocellulosic biomass, in a preferred embodiment containing typically 6 percent or more of acid detergent insoluble lignin and preferably not having any substantial alternative use in or for making human food products, is initially collected in step 102 at a convenient location (Site A) close to where the biomass is grown or produced.

Mixtures of biomasses from various sources, including biomasses from the harvesting and processing of food crops, are obviously contemplated as well and should be considered as encompassed by the use of the singular "a lignocellulosic biomass". Where several different biomasses are employed from various sources, preferably a purpose-grown non-food biomass or an agricultural waste biomass comprises the largest fraction of those several biomasses in the mixture. An example of a mixed biomass feed would be comprised of corn stover and corn fiber, with preferably corn stover comprising a greater proportion of the feed than the corn fiber. As well, a mixed biomass feed may simply be whole plant silage, for example, whole plant corn harvested and stored largely anaerobically, 'ensiled' to form silage, as most facilities for making renewable source-based chemicals, fuels and fuel additives will require year-round access to the biomass or biomass-based feeds to those facilities, in the same manner as facilities relying on petroleum-dependent feeds, and silage represents much more of a "known commodity" for the operator of such facilities than other processed biomasses.

The present invention in embodiment 100 fundamentally partially processes (at Site A) the biomass collected in step 102, in order to place the material in a condition to be more economically transported from Site A, a convenient location close to where the biomass is grown or produced, to a central processing facility ("Central Facility") located typically closer to means for making and distributing the desired biobased chemical and fuel products and closer to the customers who would ultimately purchase these chemical and fuel products. Conventionally it is expected that a Site A and the Central Facility will be 50 or more kilometers (30 miles or more) from one another, and for a number of Sites A to be on average at least the same distance removed from a Central Facility. Often, the Sites A will be on average 80 km (50 miles) or more removed from a Central Facility.

Obviously it may also be possible that the Central Facility and Site A are much closer geographically, for example, where the means for making a desired biobased chemical or fuel are already in place near a Site A - or where Site A is near a source of demand so that the embodiment of Figure 3 would ordinarily be preferred, but considerations specific to Site A (for example, zoning and permitting considerations or space limitations) prevent the embodiment of Figure 3 from being implemented.

Returning now to Figure 1, as an initial step in the process 100, biomass is collected and washed as necessary to remove dirt and other contaminants. The materials are then optionally dried, preferably to a moisture content of 10% or less. The biomass is then comminuted by any of a number of means, including without limitation by grinding, chopping and hammermilling. Depending on the biomass and on the intended downstream processing and slate of products to be produced from the various cellulosic, hemicellulosic and lignin fractions of the biomass, included preprocessing of the biomass may include separating out and recovering (through air classification or other known separatory methods) a higher protein portion of the biomass for use in animal feed and fertilizer, for example, the leaf fraction from corn stover has a greater content of nitrogen and the approximate nutritional value of hay. For a mixed biomass such as whole grain corn silage, preprocessing of the biomass may involve recovery of corn oil from the grain as an additional valued co-product.

Alternatively or additionally, and again dependent on the biomass and on the intended downstream processing and slate of products to be produced from the various cellulosic, hemicellulosic and lignin fractions of the biomass, the biomass can be preprocessed to separate out and remove portions of the whole biomass which have a comparatively high content of a species or material that will be more difficult to remove downstream and that may interfere with or make intended downstream conversions more difficult and/or may adversely affect the contemplated products from further processing. For example, a number of useful chemicals have been suggested as derivable from the pentose and/or hexose products through various catalytic transformations, and portions of the biomass may contain materials (or precursors of such materials) that would tend to deactivate a contemplated catalyst, through coking, polymerization, blocking active sites on the catalyst or other mechanisms. As well, as suggested above an intended product from the processing of the biomass may be an animal feed, and the nitrates in a lignocellulosic biomass - if more highly concentrated in a portion of the biomass that can be separated out and removed prior to further processing - would be desirably removed by preprocessing the whole biomass.

The collected or preprocessed biomass is then contacted with weak, organic acid to at least partly solubilize/depolymerize hemicellulosic materials and preferably some of the lignins in the biomass as well in weak acid step 104. Preferred acids include formic acid, malic acid, acetic acid, succinic acid and propionic acid, with formic and acetic acids being more preferred. Preferably the weak acid(s) are applied to the biomass in a hot vapor form to minimize water removal requirements in the subsequent drying step 106.

We have found that a weak acid solution of 50 percent or more in water can, with sufficient heating, be sufficient to depolymerize the hemicellulosic and lignin materials in the biomass to an extent whereby the partly depolymerized materials can serve as a binder, in effect, in the subsequent pelletization or densification step. Preferably, no additional binders are required to achieve pellets having the desired durability as further discussed below.

The weak acid can thus be applied in a preferred embodiment in the form of a hot vapor of from 50 percent acid and greater, but more preferably is applied as a concentrated vapor containing from 70 percent acid up to in excess of 90 percent acid, at a temperature of from 50 degrees Celsius to 160 degrees Celsius, a pressure of from atmospheric pressure to 3.5 MPa, gauge (500 psig), and for a residence time of at least about thirty minutes, to break down the hemicellulosic fraction and at least some of the lignins in the biomass as much as possible without compromising the durability of the subsequently-formed pellets to an undue extent whereby significant amounts of a binder must be added.

The weak acid-processed biomass then undergoes a drying or dewatering step 106 to remove sufficient moisture for allowing the dried biomass to be pelletized in step 108. The drying/dewatering step 106 can be accomplished by a number of conventional devices or combinations of such devices for concentrating an aqueous slurry and removing water therefrom to a level suited for pelletization of the remaining solids, for example, centrifuges, hydroclones, belt filter press driers, fluid bed driers, indirect or direct rotary drum driers, spin flash driers and the like. Preferably, the biomass leaving the drying step 106 will have a moisture content of 10 percent by weight or less, more preferably 8 percent or less by weight and most preferably 6 percent or less by weight to facilitate its pelletization and reduce transportation costs.

Pelletization of the solids leaving drying step 106 can be accomplished in step 108 using methods and equipment conventionally known to those skilled in the art, as pelletization of animal feeds and of woody biomass for fuels has become well-established, and will preferably result in a material with sufficient cohesiveness and integrity to withstand transport pneumatically or by conveyor belts/systems, by truck, ship or rail, or by some other means or combination of means for conveying a material from Site A to the Central Facility. For convenience, the transport of the pelletized material from Site A to a second location will be described in the claims below and elsewhere herein as "shipping" the material from a local Site A to a second location, and the use of "shipping" is not intended to be limited to ships, planes, trains or trucks or like means of vehicular transport but should be understood as inclusive of any manner in which such pelletized materials may be moved from a Site A to a second location.

In this regard, the pelletized material's needed durability - principally meaning the pellet does not produce an excessive quantity of fines in handling, transport and storage - will depend more particularly on how the material is handled, transported and stored at a given Site A, between Site A and a given Central Facility, and at the Central Facility. As well, there are several devices and related methods which have been developed for assessing pellet durability, so that precise numerical values for durability may not reasonably be assigned a priori. Preferably, however, whether by the manner in which the pelletized material is processed or by virtue of binders added to the material or both, the pelletized, partially processed biomass will be sufficiently durable so as not to experience more than five percent loss of mass by dusting or fines formation from the completion of the pelletizing step 108 to the start of solvent washing at the Central Facility, and preferably not more than three percent of the pelletized, partially processed biomass will be lost as fines in this transition.

The pelletized, partially processed biomass is then conveniently shipped to the central facility for further processing, which includes at least washing with a solvent or combination of solvents in step 110, the solvent or solvents being selected to effectively separate the at least partly depolymerized hemicellulosic materials in a product or stream 112 containing pentoses from the hydrolysis of hemicellulosic materials in the biomass, and a cellulosic solids fraction 114. The solvent wash step 110 can optionally comprise several iterations of washing and filtration, as desired.

Optionally, an additional solvent wash step 116 (in one or more iterations of washing and filtration) is employed along with the solvent wash step 110 to separate out the lignins fraction 118 of the biomass. The manner in which steps 110 and 116 are performed will depend on how the biomass has been processed at Site A, but in all cases will be conducted so as to provide a clean cellulosic pulp product or stream 114 which can then by hydrolyzed by exposure to the strong mineral acid in step 124 to yield a hexose product or stream 122.

For example, while application at a Site A of a hot, 50 percent solution of acetic acid in water, for example, is sufficient as discussed above for at least partly depolymerizing the hemicelluloses and lignins in the biomass so that the material can be pelletized and transported efficiently, the lignins in the biomass will largely not be soluble in a 50 percent solution. For step 110, a preferred approach would be to use hot water to separate the at least partly depolymerized hemicelluloses and soluble salts from the cellulosic solids fraction 114 which would also contain the water-insoluble lignins. The solvent wash step 116 would then be performed on the fraction 114 to solubilize and separate out the lignins in stream 118 and yield a material in product or stream 114 which can be acid hydrolyzed with a strong, mineral acid, a useful solvent for this purpose being a more concentrated organic acid solution applied at the requisite temperature to solubilize the lignins and separate the same from the remaining cellulosic fraction.

In an alternative embodiment wherein a more concentrated (e.g., 70 percent acid and greater versus 50 percent) weak organic acid is employed at a Site A, however, the clean cellulosic pulp 114 can be recovered in step 110 without the necessity of a further solvent wash step 116. Ethyl lactate has been found to be an effective solvent for use in step 110 in this embodiment. Other effective solvents include tetrahydrofuran, 2- methyl tetrahydrofuran, ethyl formate and ethyl acetate. The optional further solvent wash step 116 in this embodiment would be used to separate the lignins fraction 118, preferably involving washing simply with hot water to recover the water-insoluble lignins in stream 118.

Cellulosic solids fraction 114 recovered or fractionated out in this manner is then converted to a hexose product or substantially hexose stream 122 through conventional strong, mineral acid hydrolysis 124 under conditions suited to carry out this conversion. The hexose product or stream produced by the present invention in its several embodiments (as stream 122 in Figure 1, stream 220 in Figure 2 and stream 314 in Figure 3) will preferably be comprised substantially entirely of C6 monosaccharides, and of a character suited for upgrading to desired biobased chemical and fuel products with minimal further preprocessing or cleanup. Exemplary biobased chemical and fuel products which have been suggested as derivable from the C6 monosaccharides include fuel additive products through hydrogenation and hydrotreating, or ethanol, lysine, threonine, lactic, gluconic or other organic acids through fermentation.

Likewise, the pentose product or stream produced by the present invention in its several embodiments (as stream 112/120 in Figure 1, stream 208 in Figure 2, stream 316 in Figure 3) will preferably be comprised substantially entirely of C5 monosaccharides, and of a character suited for upgrading to those biobased chemical and fuel products which are derivable from such C5 monosaccharides, for example, ethanol, threonine, lysine, lactic, gluconic or other organic acids by fermentation, furfural, furfuryl alcohol, methyl tetrahydrofurfural, furfurylic acid and fuel additives generally by hydrogenation and hydrotreating.

A preferred strong, mineral acid for step 124 is sulfuric acid, applied as a 1 to 80, and preferably from 40 to 80, percent concentration aqueous sulfuric acid solution, at a temperature of from 25 degrees Celsius to 100 degrees Celsius, a pressure of from atmospheric pressure up to 0.7 MPa, gauge (100 psig), and a residence time of 15 minutes to 2 hours dependent primarily on the temperature conditions used.

The lignins fraction (as stream 118 in Figure 1, stream 212 in Figure 2 and stream 318 in Figure 3) can in like manner be put to practical further use, for example, ozonolysis to an aromatic fuel additive based, for example, on the teachings of United States Published Patent Application No. 2009/0718498A1, as a gasification feed for producing synthesis gas, as a combustion fuel, or ozonolysis to produce an aromatic sulfonation feed for producing biobased linear alkylbenzene sulfonates.

Turning now to Figure 2, an alternate embodiment 200 is shown schematically. In the embodiment 200, only the cellulosic fraction from the biomass is collected in solid form and pelletized for shipment and processing at a central facility to produce a hexose product or stream, while the hemicellulosic and lignin fractions are hydrolyzed at a first site, Site A, that is preferably convenient to where the biomass has been produced or grown. It is expected that the alternate embodiment 200 would be advantageous where the biomass has a comparatively high lignin content but no ready outlet for the products to be made from the cellulosic or lignin fractions.

More particularly, in the general embodiment 200 lignocellulosic biomass is collected and prepared for subsequent weak acid hydrolysis in step 202, in the same manner as described above for step 102 of embodiment 100. The collected and preprocessed biomass then undergoes a weak acid hydrolysis step 204, again preferably corresponding to the weak acid hydrolysis step 104 of embodiment 100. The weak acid hydrolyzed biomass, comprising at least partly depolymerized lignins and hemicellulosic materials and a cellulosic solids fraction, is in one or more iterations solvent washed and filtered in a solvent wash step 206, just as in the solvent wash step 110 described above for embodiment 100. A pentose product or stream 208 ("product" being understood herein as contemplative of a batchwise or generally discontinuous mode of operation, and "stream" being conventionally understood as referencing a continuous mode of operation) from the weak acid hydrolysis of hemicelluloses in the biomass is then optionally washed in step 210 in one or more iterations of washing and filtering, to produce a solid lignin product or stream 212 and a liquid pentose product or stream 214.

Concurrently, the cellulosic solids from the solvent wash step 206 are dried in a drying step 216, to remove moisture from the solids prior to their being pelletized in step 218 for shipment to a second venue - denominated a "Central Facility" in Figure 2. Drying step 216 and pelletizing step 218 will for embodiment 200 generally be carried out as described above for drying step 106 and pelletizing step 108 of Figure 1.

At the "Central Facility", preferably being located near a significant source of demand for a hexose product or stream 220 to be produced from the pelletized cellulosic solids from step 218, these same pelletized cellulosic solids from step 218 at a given Site A will preferably be combined with pelletized cellulosic solids from steps 218 at other localized Site A's and hydrolyzed in a strong, mineral acid hydrolysis step 222. Strong, mineral acid hydrolysis step 222 in embodiment 200 will preferably be as described above for embodiment 100, for step 124.

Figure 3, not part of the invention, may be briefly described as comprising the same basic biomass collection and preprocessing, weak acid hydrolysis, solvent washing, optional water washing and strong acid hydrolysis steps performed in common with embodiments 100 and 200, but, for example, omitting the drying and pelletization steps from embodiment 200, as the embodiment 300 is contemplated for practice at a given, localized site, ideally near where the biomass to be processed is grown or produced. Thus, the biomass is collected and preprocessed in a collection step 302, then undergoes a weak acid hydrolysis step 304 followed by a solvent wash and filtration process 306 for substantially separating the solid cellulosic fraction 308 from the solubilized/hydrolyzed hemicellulosic and lignin fractions 310. The cellulosic solids 308 are in turn hydrolyzed in a strong acid hydrolysis step 312 to yield a hexose product or stream 314, while the water-soluble pentoses and water-insoluble lignins are optionally separated and recovered as products (or streams) 316 and 318 through a wash step 320 performed on the stream 310.

Figure 3 is thus advantageously employed where the biomass to be processed is grown or produced near a source of demand for the pentoses and/or hexoses to be produced therefrom, for example, for making biobased chemicals, fuels and/or fuel additives. As an example, sugar cane - and the bagasse left in its processing to make cane sugar and related products - is grown extensively in the Mississippi River petrochemical processing corridor between Baton Rouge and New Orleans, Louisiana. Those skilled in the art will appreciate that because of the variety of lignocellulosic biomasses that can be processed numerous other similar examples can be considered wherein a sufficient supply of a suitable biomass exists near a source of demand for the pentoses and/or hexoses. In economies with well-developed chemical and fuel industries located near large population centers, opportunities for making use of figure 3 (as opposed to the embodiments 100 and 200) may be relatively more limited, while for economies with developed or developing chemical and fuel industries and fewer large population centers - or wherein the chemical and fuel industries have developed away from large population centers and nearer agrarian areas figure 3 may be preferred. In any event, those skilled in the art will be well able to determine which of the three alternatives described herein is to be preferred in a particular set of circumstances.

The alternatives 100, 200 and 300 may comprise additional steps as well, of course. It will typically be desirable, for instance, to include acid recovery and recycle for recovering and recycling for reuse either or both of the weak, organic acid(s) and the strong, mineral acid(s), and those skilled in the art will be well able to select and employ suitable means for accomplishing the separation of the acid(s) from the sugar streams in the inventive process. Acetic acid when used as the weak acid can be recovered by simple distillation, whereas formic acid forms an azeotrope with water and so requires azeotropic distillation methods when formic acid is applied as in the pulping art, as a concentrated aqueous liquid solution. Where the formic acid, however, is applied as a very concentrated vapor with small amounts of steam, the formic acid can be recovered, recycled and reused in concentrated form without the need of separating out the included water through simple distillation, preferably employing ethyl formate as an entrainer. Various methods for recovering and reusing the strong mineral acid, sulfuric acid, are described in U.S. Patent No. 5,562,777 to Farone et al. Alternatively, either or both of the weak, organic acid(s) and strong, mineral acid(s) may be simply neutralized. In the application of weak, organic acid(s) in a concentrated vapor phase, in particular, the amount of such acid employed may be insufficient to justify the expense of recovery and recycle, so that neutralization may be preferred.

A further refinement to simplify recovery of the acid used in the weak acid hydrolysis step in each of the alternatives 100, 200 and 300 would involve preparing the biomass leading into the weak acid hydrolysis step as an aqueous slurry, and providing the weak acid in a solid form that can be recovered by filtration. A zeolite would be exemplary of the types of acidic solids that could be used.

Those skilled in the art will appreciate that while preferred embodiments of the invention have been described herein, numerous variations and alternatives can in like manner be conceived. For example, an enzymatic hydrolysis can be employed in addition to or alongside the referenced acid hydrolyses to improve the efficiency of the fractionation of certain biomasses. Where silage is used for the biomass, anaerobic fermentation of the silage feed itself over a period of time produces lactic acid, and this lactic acid can be used as a weak organic acid for the initial hydrolysis either alone or in combination with weak organic acids from other sources.

## Claims

1. A method of processing a lignocellulosic biomass, in particular having an acid- detergent insoluble lignin content of 6 percent or greater by weight, on a dry weight basis, the biomass including cellulose, hemicellulose and lignin fractions, comprising the steps of:
applying a weak organic acid to the biomass to at least partly depolymerize the hemicellulosic and lignin materials in the biomass;
drying the weak-acid treated biomass to provide a sufficiently high solids content material as to be pelletized;
pelletizing the material from the drying step; and
shipping the pelletized material to a second location;
at the second location, recovering a cellulosic solids product from the pelletized material; and
contacting the cellulosic solids so isolated with a strong mineral acid to hydrolyze the same and provide a hexose product or stream.

2. A method according to claim 1 , wherein the cellulosic solids product is recovered from the pelletized material by solvent washing the pelletized material in one or more iterations with filtration, whereby the at least partly depolymerized hemicelluloses and lignin materials from the pelletized material are separated together from the remaining cellulosic solids, in particular in one or more iterations, washing the at least partly depolymerized hemicellulosic and lignin materials with filtering to separate water-insoluble lignin solids from water-soluble hemicellulosic materials.

3. A method according to claim 1, wherein the at least partly depolymerized hemicellulosic materials are first separated from the pelletized material by water washing the pelletized material one or more iterations with filtration, and the lignins are subsequently separated from the cellulosic solids product by a separate solvent wash with filtration, also in one or more iterations.

4. A method according to either of claims 2 or 3, comprising a further acid hydrolysis step conducted on the at least partly depolymerized hemicellulosic materials after the same have been separated from the lignins and cellulosic solids product, optionally further comprising fermenting the product of the further acid hydrolysis of the hemicellulosic materials to produce ethanol, lysine, threonine, lactic, gluconic or other organic acids, or hydrogenating and hydrotreating the product of the further acid hydrolysis step to yield a fuel additive product for transportation fuels.

5. A method according to either of claims 2 or 3, wherein the water- insoluble lignins are subjected to ozonolysis or exposure to one or more other oxidants, or combusted as a fuel, or supplied to a coking process for making a liquid hydrocarbon product and coke, or fed to a gasifier for producing a synthesis gas.

6. A method according to claim 1 , further comprising fermenting the hexose product or stream to produce ethanol, lysine, threonine, lactic, gluconic or other organic acids.

7. A method according to claim 1-6, wherein the lignocellulosic biomass is comprised of one or more of a mature grass, grain crop residue separately or contained in a grain silage, corn stover, wheat straw, barley straw, miscanthus specie, switchgrass, bahia grass, Sorghum specie, sugar cane bagasse, orchardgrass, reed canarygrass and cotton gin trash.

8. A method according to claim 7, wherein the lignocellulosic biomass processed comprises corn stover and corn fiber.

9. A method according to claim 8, wherein the lignocellulosic biomass processed is ensiled whole plant corn, and wherein the biomass is preprocessed before the weak organic acid is applied, preferably at a concentration of at least 50 percent, more preferably 70 percent or more, of such acid or acids in water to isolate and remove at least one component of the ensiled whole plant corn, in particular
wherein corn oil is recovered from the ensiled whole plant corn biomass before the same is contacted with the weak organic acid or
wherein the leaf fraction of corn stover is isolated and removed mechanically from the ensiled whole plant corn biomass before the same is contacted with the weak organic acid or
wherein one or more components which are higher in sulfur, nitrogen or ash content are mechanically isolated and removed from the ensiled whole plant corn biomass is contacted with the weak organic acid.

10. A method according to any of claims 1-9, wherein the weak organic acid is applied in the vapor phase at an elevated temperature of 50 degrees Celsius or greater, inparticular
wherein the weak organic acid is acetic acid or formic acid, and the acid is applied at a temperature of from 50 degrees Celsius to 160 degrees Celsius, a pressure of from atmospheric pressure to 3.5 MPa, gauge, and for a time period of 30 minutes and more.

11. A method according to any of claims 1-10, wherein the weak acid- treated biomass is dried to a moisture content of 10 percent or less by weight, in particular wherein the dried biomass is pelletized without the addition of a further binder.

12. A method according to any of claims 1-11, wherein the weak acid treatment, drying and pelletizing steps are applied at a plurality of locations for shipment of pelletized material to a common second location.

13. A method according to claim 1, further comprising preprocessing the biomass before contact with the weak organic acid to isolate and remove at least one component of the biomass that is higher in protein and suitable for use in or for an animal feed, or that has a higher than desired sulfur, nitrogen or ash content.

14. A method of processing a lignocellulosic biomass including cellulose, hemicellulose and lignin fractions, comprising the steps of:
applying a weak organic acid to the biomass to hydrolyze hemicellulosic and lignin materials in the biomass;
in one or more iterations, washing the weak acid-treated material with a solvent or solvent mixture and filtering to separate hemicellulosic and lignin materials in the filtrate and cellulosic materials as a solid residue;
drying the solid residue to provide a sufficiently high solids content material as to be pelletized;
pelletizing the material from the drying step;
shipping the pelletized material to a second location; and at the second location, contacting the cellulosic materials so isolated with a strong mineral acid to hydrolyze the same and provide a hexose product or stream.

15. A method according to claim 14, wherein the weak organic acid is applied in the vapor phase at an elevated temperature of 50 degrees Celsius and greater, in particular wherein the weak organic acid is acetic acid or formic acid, and the acid is applied at a temperature of from 50 degrees Celsius to 160 degrees Celsius, a pressure of from atmospheric pressure to 3.5 MPa, gauge, and for a time period of 30 minutes and more or
wherein the weak organic acid or acids are applied to the lignocellulosic biomass at a concentration of at least 50 percent, in particular of 70 percent or more of such acid or acids in water.

## Patentansprüche

1. Verfahren zur Verarbeitung einer lignocellulosischen Biomasse, die insbesondere einen Gehalt an in Säure/Tensid unlöslichem Lignin auf Trockengewichtsbasis von 6 Gew.-% oder mehr aufweist, wobei die Biomasse eine Cellulose-, eine Hemicellulose- und eine Ligninfraktion umfasst, umfassend die Schritte:
Anwenden einer schwachen organischen Säure auf die Biomasse, so dass das Hemicellulose- und das Ligninmaterial in der Biomasse wenigstens teilweise depolymerisieren;
Trocknen der mit schwacher Säure behandelten Biomasse, bis man ein Material mit einem zum Granulieren ausreichend hohen Feststoffgehalt erhält;
Granulieren des Materials aus dem Trocknungsschritt; und
Transportieren des granulierten Materials an einen zweiten Standort;
Gewinnen eines cellulosischen Feststoffs als Produkt aus dem granulierten Material an dem zweiten Standort; und
In-Kontakt-Bringen des so isolierten cellulosischen Feststoffs mit einer starken Mineralsäure zum Hydrolysieren desselben, wobei man ein Hexoseprodukt oder einen Hexoseproduktstrom erhält.

2. Verfahren gemäß Anspruch 1, wobei der cellulosische Feststoff als Produkt dadurch aus dem granulierten Material gewonnen wird, dass man das granulierte Material in einer oder mehreren Iterationen mit Filtration mit einem Lösungsmittel wäscht, wodurch die wenigstens teilweise depolymerisierten Hemicellulose- und Ligninmaterialien aus dem granulierten Material zusammen aus dem übrigen cellulosischen Feststoff abgetrennt werden, insbesondere in einer oder mehreren Iterationen, Waschen der wenigstens teilweise depolymerisierten Hemicellulose- und Ligninmaterialien mit Filtration, wobei wasserunlöslicher Ligninfeststoff von wasserlöslichen hemicellulosischen Materialien abgetrennt wird.

3. Verfahren gemäß Anspruch 1, wobei die wenigstens teilweise depolymerisierten Hemicellulosematerialien zuerst durch Waschen des granulierten Materials mit Wasser über eine oder mehrere Iterationen mit Filtration aus dem granulierten Material abgetrennt werden und die Lignine anschließend durch eine getrennte Waschung mit Lösungsmittel mit Filtration, ebenfalls in einer oder mehreren Iterationen, aus dem cellulosischen Feststoff als Produkt abgetrennt werden.

4. Verfahren gemäß einem der Ansprüche 2 oder 3, umfassend einen weiteren Säurehydrolyseschritt, der mit den wenigstens teilweise depolymerisierten Hemicellulosematerialien durchgeführt wird, nachdem diese von den Ligninen und dem cellulosischen Feststoff als Produkt abgetrennt wurden, gegebenenfalls weiterhin umfassend das Fermentieren des Produkts der weiteren Säurehydrolyse der Hemicellulosematerialien, wobei Ethanol, Lysin, Threonin, Milchsäure, Gluconsäure oder andere organische Säuren entstehen, oder Hydrieren und Hydrotreating des Produkts des weiteren Säurehydrolyseschritts, wobei man ein Kraftstoffadditivprodukt für Kraftstoffe erhält.

5. Verfahren gemäß einem der Ansprüche 2 oder 3, wobei die wasserunlöslichen Lignine einer Ozonolyse unterzogen oder einem oder mehreren anderen Oxidantien ausgesetzt oder als Brennstoff verbrannt oder einem Verkokungsprozess zugeführt werden, um ein flüssiges Kohlenwasserstoffprodukt und Koks herzustellen, oder einem Vergaser zugeführt werden, um ein Synthesegas herzustellen.

6. Verfahren gemäß Anspruch 1, weiterhin umfassend das Fermentieren des Hexoseprodukts oder Hexoseproduktstroms, wobei Ethanol, Lysin, Threonin, Milchsäure, Gluconsäure oder andere organische Säuren entstehen.

7. Verfahren gemäß Anspruch 1 bis 6, wobei die lignocellulosische Biomasse aus einem oder mehreren aus einem reifen Gras, Getreideernterest getrennt oder in einer Getreidesilage enthalten, Maisstroh, Weizenstroh, Gerstestroh, Elefantengras, Rutenhirse, Bahiagras, Sorghum, Zuckerrohrbagasse, Knaulgras, Rohrglanzgras und Egreniermaschinenabfällen besteht.

8. Verfahren gemäß Anspruch 7, wobei die verarbeitete lignocellulosische Biomasse Maisstroh und Maisfasern umfasst.

9. Verfahren gemäß Anspruch 8, wobei es sich bei der verarbeiteten lignocellulosischen Biomasse um silierten Vollpflanzenmais handelt und wobei die Biomasse vorverarbeitet wird, bevor die schwache organische Säure angewendet wird, vorzugsweise mit einer Konzentration von wenigstens 50 Prozent, besonders bevorzugt 70 Prozent oder mehr, einer solchen Säure oder solcher Säuren in Wasser, um wenigstens eine Komponente des silierten Vollpflanzenmaises zu isolieren und zu entfernen, wobei insbesondere
Maisöl aus der silierten Vollpflanzenmais-Biomasse gewonnen wird, bevor dieselbe mit der schwachen organischen Säure in Kontakt gebracht wird, oder
die Blätterfraktion des Maisstrohs mechanisch aus der silierten Vollpflanzenmais-Biomasse isoliert und entfernt wird, bevor dieselbe mit der schwachen organischen Säure in Kontakt gebracht wird, oder
eine oder mehrere Komponenten, die einen höheren Schwefel-, Stickstoff- oder Aschegehalt aufweisen, mechanisch aus der silierten Vollpflanzenmais-Biomasse isoliert und entfernt werden, bevor dieselbe mit der schwachen organischen Säure in Kontakt gebracht wird.

10. Verfahren gemäß einem der Ansprüche 1 bis 9, wobei die schwache organische Säure in der Dampfphase bei einer erhöhten Temperatur von 50 °C oder mehr angewendet wird, wobei es sich insbesondere bei der schwachen organischen Säure um Essigsäure oder Ameisensäure handelt und die Säure bei einer Temperatur von 50 °C bis 160 °C, einem Manometerdruck von Atmosphärendruck bis 3,5 MPa und während einer Zeitspanne von 30 Minuten und mehr angewendet wird.

11. Verfahren gemäß einem der Ansprüche 1 bis 10, wobei die mit einer schwachen Säure behandelte Biomasse auf einen Feuchtigkeitsgehalt von 10 Gew.-% oder weniger getrocknet wird, wobei insbesondere die getrocknete Biomasse ohne Zugabe eines weiteren Bindemittels granuliert wird.

12. Verfahren gemäß einem der Ansprüche 1 bis 11, wobei die Schritte der Behandlung mit schwacher Säure, Trocknung und Granulierung an einer Vielzahl von Standorten zum Transport von granuliertem Material zu einem gemeinsamen zweiten Standort angewendet werden.

13. Verfahren gemäß Anspruch 1, weiterhin umfassend die Vorverarbeitung der Biomasse vor dem Kontakt mit der schwachen organischen Säure, um wenigstens eine Komponente der Biomasse, die einen höheren Proteingehalt aufweist und für die Verwendung in oder als Tierfutter geeignet ist oder die einen Schwefel-, Stickstoff- oder Aschegehalt aufweist, der höher als erwünscht ist, zu isolieren und zu entfernen.

14. Verfahren zur Verarbeitung einer lignocellulosischen Biomasse, die eine Cellulose-, eine Hemicellulose- und eine Ligninfraktion umfasst, umfassend die Schritte:
Anwenden einer schwachen organischen Säure auf die Biomasse, so dass das Hemicellulose- und das Ligninmaterial in der Biomasse hydrolysieren;
Waschen des mit einer schwachen Säure behandelten Materials in einer oder mehreren Iterationen mit einem Lösungsmittel oder Lösungsmittelgemisch und Filtrieren, um Hemicellulose- und Ligninmaterial im Filtrat und Cellulosematerialien als festen Rückstand voneinander zu trennen;
Trocknen des festen Rückstands, bis man ein Material mit einem zum Granulieren ausreichend hohen Feststoffgehalt erhält;
Granulieren des Materials aus dem Trocknungsschritt;
Transportieren des granulierten Materials an einen zweiten Standort; und an dem zweiten Standort, In-Kontakt-Bringen der so isolierten Cellulosematerialien mit einer starken Mineralsäure zum Hydrolysieren desselben, wobei man ein Hexoseprodukt oder einen Hexoseproduktstrom erhält.

15. Verfahren gemäß Anspruch 14, wobei die schwache organische Säure in der Dampfphase bei einer erhöhten Temperatur von 50 °C und mehr angewendet wird, wobei es sich insbesondere bei der schwachen organischen Säure um Essigsäure oder Ameisensäure handelt und die Säure bei einer Temperatur von 50 °C bis 160 °C, einem Manometerdruck von Atmosphärendruck bis 3,5 MPa und während einer Zeitspanne von 30 Minuten und mehr angewendet wird oder
wobei die schwache organische Säure oder die schwachen organischen Säuren mit einer Konzentration von wenigstens 50 Prozent, insbesondere 70 Prozent oder mehr, einer solchen Säure oder solcher Säuren in Wasser auf die lignocellulosische Biomasse angewendet werden.

## Revendications

1. Procédé de traitement d'une biomasse lignocellulosique, en particulier présentant une teneur en lignine insoluble dans un détergent acide de 6 pour cent, ou plus, en poids, sur la base d'un poids à sec, la biomasse comportant de la cellulose, de l'hémicellulose et des fractions de lignine, comprenant les étapes de :
application d'un acide organique faible à la biomasse pour, au moins partiellement, dépolymériser les matériaux hémicellulosiques et de lignine dans la biomasse ;
séchage de la biomasse traitée par l'acide faible afin de fournir un matériau à teneur en solides suffisamment élevée de façon à le transformer en granulés ;
transformation en granulés du matériau obtenu à l'étape de séchage ; et
transport du matériau en granulés vers un second site ;
au niveau du second site, récupération d'un produit de matières solides cellulosiques à partir du matériau en granulés ; et
mise en contact des matières solides cellulosiques ainsi isolées avec un acide minéral fort pour les hydrolyser et fournir un produit ou un courant d'hexose.

2. Procédé selon la revendication 1, dans lequel le produit de matières solides cellulosiques est récupéré à partir du matériau en granulés en lavant au solvant le matériau en granulés en une ou en plusieurs itération(s) avec filtration, de sorte que les hémicelluloses, au moins partiellement dépolymérisées, et les matériaux de lignine provenant du matériau en granulés sont séparés ensemble des matières solides cellulosiques restantes, en particulier en une ou plusieurs itération(s), en lavant les matériaux hémicellulosiques au moins partiellement dépolymérisés et les matériaux de lignine , avec filtration pour séparer les matières solides de lignine insolubles dans l'eau des matériaux hémicellulosiques solubles dans l'eau.

3. Procédé selon la revendication 1, dans lequel les matériaux hémicellulosiques, au moins partiellement dépolymérisés, sont d'abord séparés du matériau en granulés en lavant à l'eau le matériau en granulés en une ou plusieurs itération(s) avec filtration, et les lignines sont ensuite séparées du produit de matières solides cellulosiques par un lavage au solvant séparé avec filtration, réalisé également en une ou plusieurs itération(s).

4. Procédé selon l'une ou l'autre des revendications 2 ou 3, comportant une étape supplémentaire d'hydrolyse à l'acide réalisée sur les matériaux hémicellulosiques, au moins partiellement dépolymérisés, après que ceux-ci ont été séparés des lignines et du produit de matières solides cellulosiques, comprenant, de plus, facultativement, de faire fermenter le produit de l'hydrolyse acide supplémentaire des matériaux hémicellulosiques afin de produire de l'éthanol, de la lysine, de la thréonine, des acides lactiques, gluconiques ou d'autres acides organiques, ou d'hydrogéner et d'hydrotraiter le produit de l'étape supplémentaire d'hydrolyse acide pour aboutir à un produit additif pour carburant destiné aux carburants de transport.

5. Procédé selon l'une ou l'autre des revendications 2 ou 3, dans lequel les lignines insolubles dans l'eau sont soumises à une ozonolyse ou à une exposition à un ou à plusieurs autre(s) oxydant(s), ou sont brûlés en tant que combustible, ou fournis à un processus de carbonisation pour produire un produit d'hydrocarbure liquide et du coke, ou sont fournis à un gazéificateur pour produire un gaz de synthèse.

6. Procédé selon la revendication 1, comprenant, de plus, le fait de faire fermenter le produit ou le courant d'hexose afin de produire de l'éthanol, de la lysine, de la thréonine, de l'acide lactique, de l'acide gluconique ou d'autres acides organiques.

7. Procédé selon les revendications 1 à 6, dans lequel la biomasse lignocellulosique est constituée de l'un ou de plusieurs composant(s) d'herbe haute, de résidus de cultures céréalières fournis séparément ou contenus dans un ensilage de céréales, de canne de maïs, de paille de blé, de paille d'orge, de miscanthus, de panic érigé, d'herbe Bahia, de sorgho, de bagasse de canne à sucre, de dactyle aggloméré, d'alpiste roseau des Canaries et de déchets de l'égrenage du coton.

8. Procédé selon la revendication 7, dans lequel la biomasse lignocellulosique traitée comporte de la canne de maïs et de la fibre de maïs.

9. Procédé selon la revendication 8, dans lequel la biomasse lignocellulosique traitée est du maïs ensilé sous forme de plante complète et dans lequel la biomasse est prétraitée avant que l'acide organique faible ne soit appliqué, de préférence, à une concentration d'au moins 50 pour cent, avec plus de préférence de 70 pour cent ou davantage, de cet acide ou de ces acides dans l'eau pour isoler et éliminer au moins un composant du maïs ensilé sous forme de plante complète, en particulier,
dans lequel, de l'huile de maïs est récupérée à partir de la biomasse du maïs ensilé sous forme de plante complète avant que celle-ci ne soit mise en contact avec l'acide organique faible ou
dans lequel la fraction feuille de la canne de maïs est isolée et éliminée mécaniquement de la biomasse du maïs ensilé sous forme de plante complète avant que celle-ci ne soit mise en contact avec l'acide organique faible ou dans lequel un ou plusieurs composant(s) qui a (ont) une teneur plus élevée en soufre, azote ou en cendres est (sont) isolé(s) mécaniquement et éliminé(s) de la biomasse de maïs ensilé sous forme de plante complète avant que celle-ci ne soit mise en contact avec l'acide organique faible.

10. Procédé selon l'une quelconque des revendications 1 à 9, dans lequel l'acide organique faible est appliqué en phase vapeur à une température élevée de 50 degrés Celsius ou plus, en particulier,
dans lequel, l'acide organique faible est de l'acide acétique ou de l'acide formique, et dans lequel l'acide est appliqué à une température allant de 50 degrés Celsius à 160 degrés Celsius, sous une pression de jauge allant de la pression atmosphérique à 3,5 Mpa et pendant une durée de 30 minutes et davantage.

11. Procédé selon l'une quelconque des revendications 1 à 10, dans lequel la biomasse traitée à l'acide faible est séchée jusqu'à une teneur en humidité de 10 pour cent ou moins en poids, dans lequel, en particulier, la biomasse séchée est transformée en granulés sans l'adjonction d'un liant supplémentaire.

12. Procédé selon l'une quelconque des revendications 1 à 11, dans lequel les étapes de traitement à l'acide faible, de séchage et de mise en granulés sont appliquées au niveau d'une pluralité de sites pour le transport du matériau en granulés vers un second site commun.

13. Procédé selon la revendication 1, comportant, de plus, de prétraiter la biomasse avant de la mettre en contact avec l'acide organique faible afin d'isoler et d'éliminer au moins un composant de la biomasse qui est plus riche en protéines et qui est approprié pour être utilisé dans, ou pour, un aliment pour animaux, ou qui présente une teneur en soufre, en azote ou en cendres plus élevée que souhaitée.

14. Procédé de traitement d'une biomasse lignocellulosique comportant de la cellulose, de l'hémicellulose et des fractions de lignine, comportant les étapes de :
application d'un acide organique faible à la biomasse pour hydrolyser des matériaux hémicellulosiques et de lignine dans la biomasse ;
en une ou plusieurs itération(s), lavage du matériau traité à l'acide faible avec un solvant ou un mélange de solvant et filtrer pour séparer les matériaux hémicellulosiques et les matériaux de lignine dans le filtrat et les matériaux cellulosiques sous la forme de résidu solide ;
séchage du résidu solide pour fournir un matériau à teneur en matières solides suffisamment élevée pour être mis sous forme de granulés ;
mettre sous forme de granulés le matériau obtenu à l'étape de séchage ;
transport du matériau en granulés vers un second site ; et au niveau du second site, mise en contact des matériaux cellulosiques ainsi isolés avec un acide minéral fort afin de les hydrolyser et de fournir un produit ou un courant d'hexose.

15. Procédé selon la revendication 14, dans lequel l'acide organique faible est appliqué en phase vapeur à une température élevée de 50 degrés Celsius etdavantage, dans lequel, en particulier, l'acide organique faible est de l'acide acétique ou de l'acide formique et dans lequel l'acide est appliqué à une température allant de 50 degrés Celsius à 160 degrés Celsius, sous une pression de jauge allant de la pression atmosphérique à 3,5 MPa et pendant une durée de 30 minutes et davantage ou
dans lequel l'acide ou les acides organique(s) faible(s) est (sont) appliqué(s) à la biomasse lignocellulosique à une concentration d'au moins 50 pour cent, en particulier de 70 pour cent, ou davantage, dudit acide ou desdits acides dans l'eau.
